(19) Europäisches Patentamt
European Patent Office
Office européen des brevets

(11) Numéro de publication: **0 315 539 B1**

(12) **FASCICULE DE BREVET EUROPEEN**

(45) Date de publication de fascicule du brevet: **26.10.94**

(51) Int. Cl.$^5$: **C07H 15/16**, C07H 17/08, A61K 31/70, A61K 7/00

(21) Numéro de dépôt: **88402770.7**

(22) Date de dépôt: **03.11.88**

(54) **Esters aromatiques polycycliques d'antibiotiques macrolidiques et lincosamidiques, leur procédé de préparation et compositions pharmaceutiques et cosmétiques les contenant.**

(30) Priorité: **04.11.87 LU 87040**

(43) Date de publication de la demande:
**10.05.89 Bulletin 89/19**

(45) Mention de la délivrance du brevet:
**26.10.94 Bulletin 94/43**

(84) Etats contractants désignés:
**AT BE CH DE ES FR GB GR IT LI NL SE**

(56) Documents cités:
**EP-A- 0 210 929**
**FR-A- 2 169 104**
**FR-A- 2 598 420**
**GB-A- 2 175 303**

(73) Titulaire: **L'OREAL**
**14, Rue Royale**
**F-75008 Paris (FR)**

(72) Inventeur: **Philippe, Michel**
**3, rue de l'Aubépine**
**F-92160 Antony (FR)**
Inventeur: **Sebag, Henri**
**26, rue Erlanger**
**F-75016 Paris (FR)**

(74) Mandataire: **Stalla-Bourdillon, Bernard et al**
**CABINET NONY & CIE**
**29, rue Cambacérès**
**F-75008 Paris (FR)**

EP 0 315 539 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet des esters aromatiques polycycliques d'antibiotiques macrolidiques et lincosamidiques, leur procédé de préparation et les compositions pharmaceutiques et cosmétiques les contenant dans le traitement de diverses dermatoses, notamment dans le traitement de l'acné.

Plus particulièrement, les esters selon l'invention sont destinés au traitement des dermatoses infectieuses ou non.

Dans le traitement de l'acné, l'érythromycine parmi les macrolides ainsi que la clindamycine parmi les lincosamides ont été plus particulièrement préconisées, mais leur emploi nécessite (notamment pour l'érythromycine) des concentrations relativement élevées en vue d'obtenir une action satisfaisante.

Par ailleurs le traitement par ces antibiotiques s'est avéré dans certains cas peu efficace, dans la mesure où certaines souches de propionibacterium acnes ont présenté une résistance progressive à leur égard.

L'application topique de clindamycine et plus particulièrement d'érythromycine se heurte par ailleurs à un problème de pénétration à travers le stratum cornéum limitant de ce fait leur efficacité.

Les esters d'antibiotiques selon l'invention apportent une solution satisfaisante au problème soulevé par l'utilisation de ces antibiotiques dans le traitement de l'acné, dans la mesure où il s'est avéré que ceux-ci avaient une action sur propionibacterium acnes, principal germe responsable des phénomènes d'inflammation de la peau.

Les esters aromatiques polycycliques selon l'invention ont du fait de leur structure un caractère lipophile prononcé, ce qui facilite une meilleure pénétration à travers l'épiderme.

Les nouveaux esters selon l'invention sont bien tolérés par la peau et se sont révélés être beaucoup moins toxiques par voie orale que l'association antibiotique/acide.

Par ailleurs ils présentent par rapport aux esters connus l'avantage de posséder une activité comédolytique potentielle due à la chaîne acide correspondante, ce qui confère à ces esters une image de "prodrug".

L'état de la technique relatif aux esters de macrolides est représenté notamment par le brevet français n° 85.07287 (2.582.000) qui se rapporte à des esters gras polyinsaturés d'érythromycine A tels que le linoléate et le linolénate d'érythromycine A.

L'état de la technique relatif aux esters de lincosamides est représenté notamment par le brevet allemand n° 2.017.003 qui décrit la préparation d'esters de lincomycine et de clindamycine dont la chaîne acyle est comprise entre 1 et 18 atomes de carbone.

La présente invention a pour objet à titre de produit industriel nouveau, des esters aromatiques polycycliques de macrolides et de lincosamides, ceux-ci correspondant à la forme générale suivante:

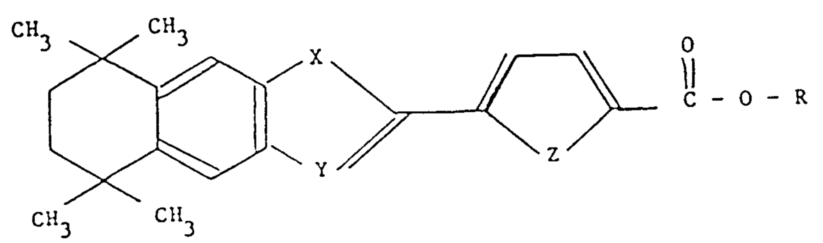

(I)

dans laquelle:

R représente un radical dérivé d'un macrolide ou d'un lincosamide,

Y représente CH ou un atome d'azote

- quand Y représente CH , X représente un radical vinylène (-CH = CH-), un atome d'oxygène, un atome de soufre ou $NR_1$ ,
- quand Y représente un atome d'azote, X représente un atome d'oxygène, un atome de soufre ou NH , et

Z représente un radical vinylène, un atome d'oxygène, un atome de soufre ou $NR_2$ ,

$R_1$ et $R_2$ représentant un atome d'hydrogène ou un radical méthyle, et les mélanges et sels desdits esters.

Parmi les macrolides on peut citer l'érythromycine A, la roxythromycine, l'oléandomycine, la josamycine et les spiramycines I, II et III.

Parmi les lincosamides on peut citer la lincomycine et la clindamycine.

A - Les esters d'érythromycine A et de roxythromycine peuvent être représentés par la formule:

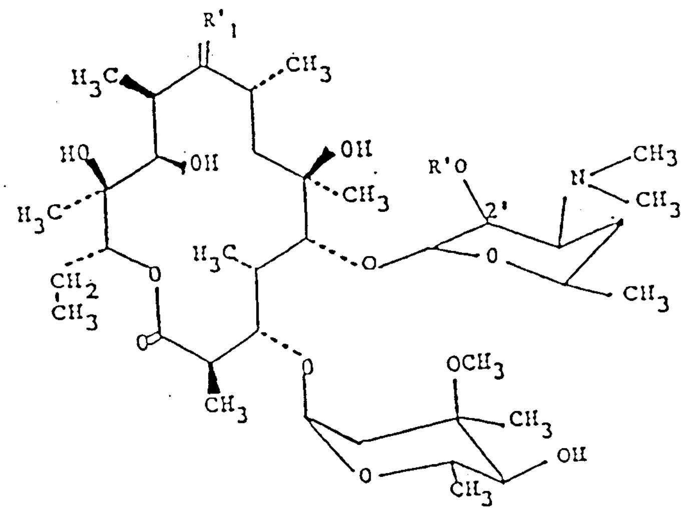

(II)

dans laquelle:

$R'_1$ représente O (érythromycine A) ou N$\sim$O-$CH_2$-O-$CH_2$-$CH_2$-O-$CH_3$ (roxythromycine),

R′ représente le radical acyle suivant:

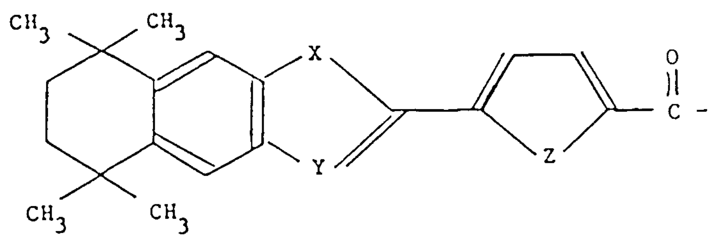

( I ' )

X, Y et Z ayant les mêmes significations que celles données ci-dessus.

Ces esters d'érythromycine A et de roxythromycine sont ceux en position 2′.

B - Les esters d'oléandomycine peuvent être représentés par la formule suivante:

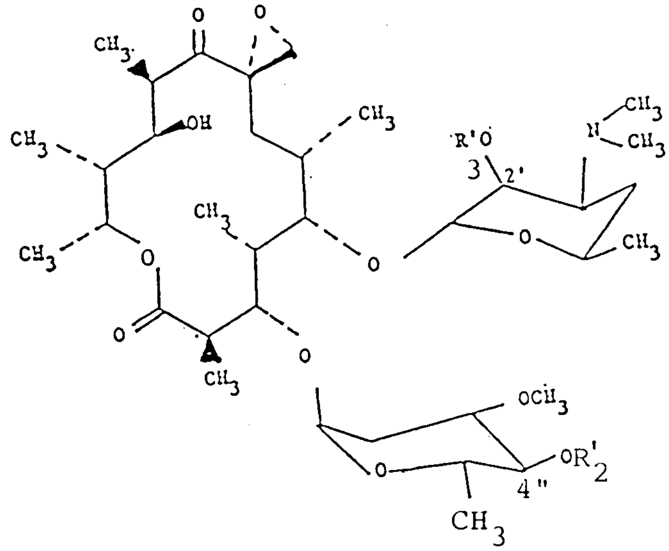

(III)

dans laquelle:

$R'_2$ ou $R'_3$ représente R′ ou un atome d'hydrogène, étant entendu que l'un au moins représente R′, R′ ayant la même signification que ci-dessus.

Ces esters sont également ceux en position 2′ et/ou 4″, mais ils peuvent se présenter sous forme d'un mélange.

C - Les esters de josamycine peuvent être représentés par la formule suivante:

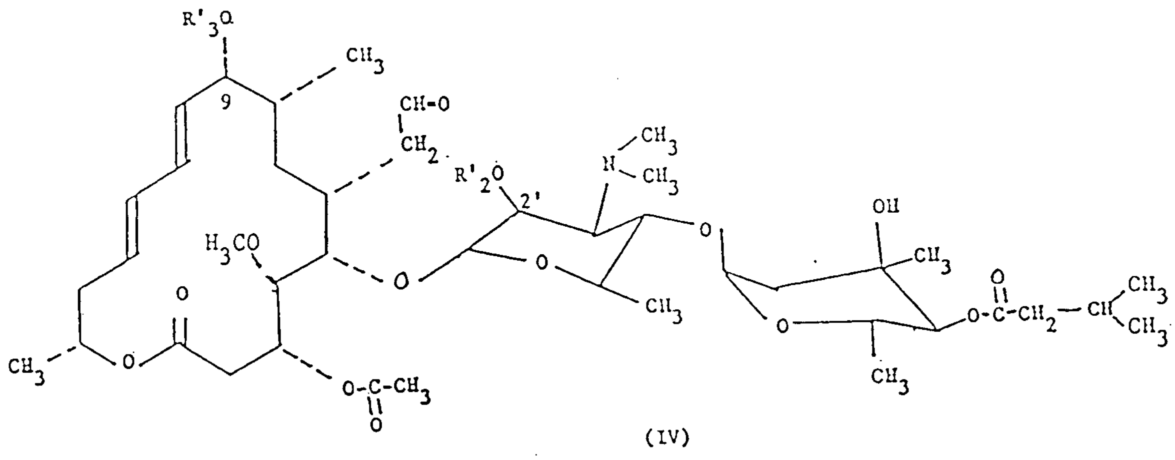

(IV)

dans laquelle:

$R'_2$ ou $R'_3$ représente R′ ou un atome d'hydrogène étant entendu que l'un au moins représente R′, R′ ayant la même signification que ci-dessus.

Ces esters sont ceux en position 9 et/ou 2′, mais ils peuvent se présenter sous forme d'un mélange.

D - Les esters des spiramycines peuvent être représentés par la formule suivante:

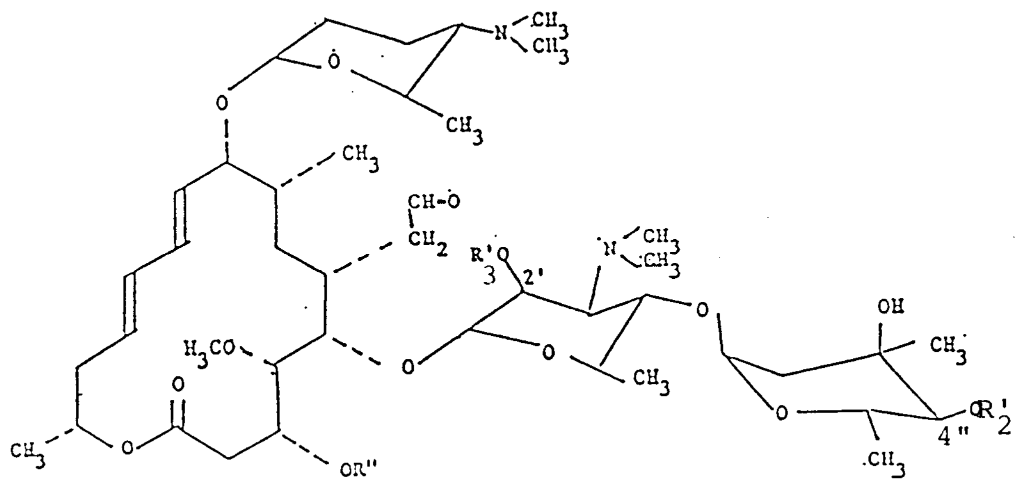

dans laquelle:

$R'_2$ ou $R'_3$ représente R′ ou un atome d'hydrogène, étant entendu que l'un au moins représente R′ R′ ayant la même signification que ci-dessus,

et R″ représente un atome d'hydrogène (spiramycine I), un radical acétyl (spiramycine II) ou un radical propionyl (spiramycine III).

Ces esters des spiramycines (I), (II) et (III) sont ceux en position 2′ et/ou 4″, ceux-ci pouvant éventuellement se présenter sous forme d'un mélange.

E - Les esters de lincomycine et de clindamycine peuvent être représentés respectivement par les formules (VI) et (VII) suivantes:

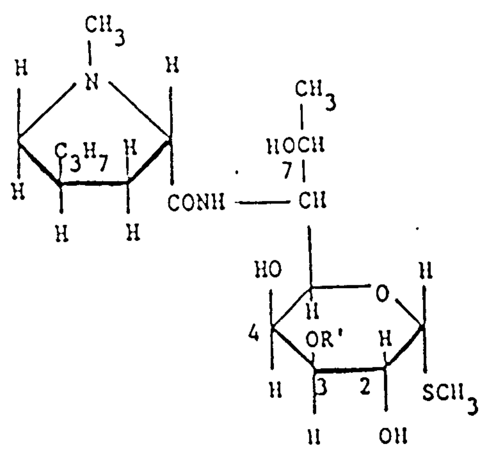

(VI)

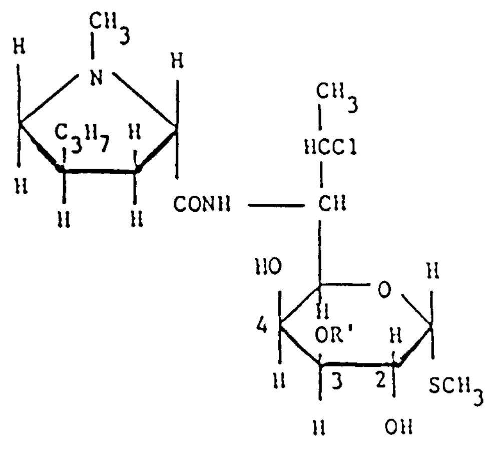

(VII)

dans lesquels:

R′ a la même signification que celle donnée ci-dessus.

Les esters de lincomycine (VI) et de clindamycine (VII) sont de préférence ceux en position 3. Toutefois ils peuvent se présenter sous forme de mélanges avec les esters en position 2, 4 et 7 de lincomycine et avec les esters en position 2 et 4 de clindamycine.

Parmi les esters aromatiques polycycliques d'antibiotiques de formule (I) selon l'invention on peut notamment mentionner les suivants:

O-[(tétrahydro-5,6,7,8-tétraméthyl-5,5,8,8-anthracényl-2)-5furanne-carbonyl-2]-2′-érythromycine A,

O-[(tétrahydro-5,6,7,8-tétraméthyl-5,5,8,8-anthracényl-2)-5furanne-carbonyl-2]-2′-oléandomycine,

O-[(tétrahydro-5,6,7,8-tétraméthyl-5,5,8,8-anthracényl-2)-5furanne-carbonyl-2]-3-clindamycine,

O-[(tétrahydro-5,6,7,8-tétraméthyl-5,5,8,8-anthracényl-2)-5furanne-carbonyl-2]-2′-roxythromycine,

O-[(tétrahydro-5,6,7,8-tétraméthyl-5,5,8,8-anthracényl-2)-5furanne-carbonyl-2]-9-josamycine et son isomère en 2′,

O-[(tétrahydro-5,6,7,8-tétraméthyl-5,5,8,8-naphto [2,3-b] furanne-yl-2)-4- benzoyl]-2′ -érythromycine A,

O-[(tétrahydro-5,6,7,8-tétraméthyl-5,5,8,8-naphto[2,3-b] thiényl-2)-4- benzoyl]-2′-érythromycine A,

O-[(tétrahydro-2,3,5,6-tétraméthyl-1,1,4,4-1H-benz[f] indolyl-2)-4- benzoyl]-2′-roxythromycine,

O-[ (méthyl-1-tétrahydro-2,3,5,6- tétraméthyl-1,1,4,4,-1H-benz[f]indolyl-2)-4- benzoyl]-9-josamycine et son isomère en 2′,

O-[(tétrahydro-5,6,7,8-tétraméthyl-5,5,8,8-naphto[2,3-d] oxazolyl-2)-4- benzoyl]-2′-oléandomycine,

O-[(tétrahydro-5,6,7,8-tétraméthyl-5,5,8,8-naphto[2,3-d] imidazolyl-2)-4- benzoyl]-2′ -érythromycine A,

O-[(tétrahydro-5,6,7,8-tétraméthyl-5,5,8,8-anthracényl-2)-4-benzoyl]-3 -lincomycine,

O-[(tétrahydro-5,6,7,8-tétraméthyl-5,5,8,8-anthracényl-2)-5-thiophène-carbonyl-2]-2′-spiramycine (I), (II) et (III).

O-[(tétrahydro-5,6,7,8-tétraméthyl-5,5,8,8-naphto[2,3-b] furanne-yl-2)-4-benzoyl]-2′-oléandomycine.

La présente invention a également pour objet le procédé de préparation des esters aromatiques polycycliques d'antibiotiques de formule (I) selon l'invention.

Différents procédés d'estérification peuvent être utilisés mais de préférence cette estérification est réalisée en milieu solvant organique anhydre, de préférence dans le tétrahydrofuranne seul ou en mélange avec un autre solvant organique comme la pyridine, en faisant réagir un excès d'anhydride mixte de formule:

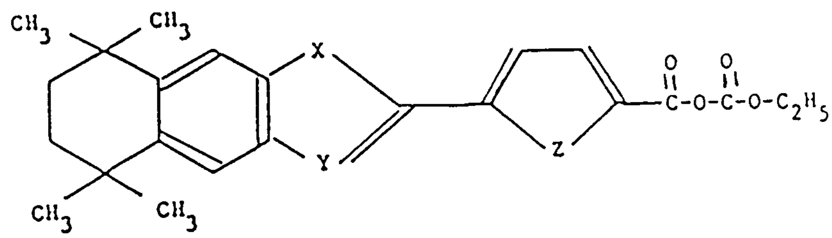

dans laquelle:

X, Y et Z ont les mêmes significations que ci-dessus, ledit anhydride étant préparé in situ, (par exemple à partir de chloroformiate d'éthyle et de l'acide correspondant) avec un macrolide ou lincosamide, sous forme de base, en présence d'une base organique ou minérale comme la pyridine et/ou l'hydrogénocarbonate de sodium.

Cette méthode à l'anhydride mixte permet d'obtenir préférentiellement les esters en position 2′ des macrolides et/ou en position 9 notamment pour la josamycine et/ou en position 4″ notamment pour les spiramycines et en position 3 des lincosamides dans de bonnes conditions de rendement.

Les autres procédés d'estérification, notamment de lincomycine et de clindamycine par la méthode utilisant les imidazolides des acides correspondants dans un solvant anhydre comme le N,N-diméthylformamide en présence d'une base comme le tertiobutylate de sodium ou de potassium, conduisent à un mélange d'esters de ces antibiotiques.

La présente invention a également pour objet des compositions pharmaceutiques administrables par voie topique, orale, parentérale ou rectale ainsi que des compositions à caractère cosmétique pour le traitement de diverses dermatoses, notamment l'acné, ces compositions se présentant sous forme anhydre et contenant au moins un ester selon l'invention, tel que défini ci-dessus, à une concentration comprise entre 0,001 et 10% mais de préférence entre 0,01 et 1% en poids par rapport au poids total de la composition.

Pour la préparation des compositions selon l'invention contenant, comme constituant actif, au moins un ester selon l'invention tel que défini ci-dessus, on peut faire appel à des véhicules et adjuvants décrits dans la littérature pour la pharmacie, la cosmétique et les domaines apparentés.

Pour la préparation des solutions, on peut utiliser par exemple un (ou des) solvant(s) organique(s) acceptable(s) d'un point de vue physiologique.

Les solvants organiques acceptables sont pris notamment dans le groupe constitué par l'acétone, l'alcool isopropylique, les triglycérides d'acides gras, les esters d'alkyle en $C_1$-$C_4$ d'acides à courte chaîne, les éthers du polytétrahydrofuranne et les silicones comme les cyclométhicones.

Les compositions selon l'invention peuvent également renfermer un agent épaississant tel qu'un dérivé de la cellulose à raison de 0,5 à 20% en poids par rapport au poids total de la composition.

Les compositions selon l'invention peuvent en outre contenir en association avec au moins un ester selon l'invention, au moins un autre agent anti-acnéique connu.

On peut, si nécessaire, ajouter un adjuvant usuel pris dans le groupe formé par les agents anti-oxydants, les agents conservateurs, les parfums et les colorants.

Parmi les anti-oxydants utilisables, on citera par exemple la t-butylhydroxyquinone, le butylhydroxyanisole, le butylhydroxytoluène et l'α-tocophérol et ses dérivés.

Les transformations pharmacologiques et galéniques des composés selon l'invention s'effectuent de façon connue.

Les formes galéniques peuvent être pour la voie topique des crèmes, des laits, des gels, des lotions plus ou moins épaissies, des lotions portées par des tampons, des pommades, des sticks ou bien des formulations aérosols se présentant sous forme de sprays ou de mousses.

Les compositions par voie orale peuvent se présenter sous forme de comprimés, de gelules, de dragées, de sirops, de suspensions, d'émulsions, de poudres, de granulés ou de solutions.

Les compositions peuvent également se présenter sous forme de suppositoires.

Le traitement de l'acné à l'aide des compositions topiques selon l'invention consiste à appliquer, deux ou trois fois par jour, une quantité suffisante sur les zones de la peau à traiter et ceci pendant une période de temps de 6 à 30 semaines et de préférence de 12 à 24 semaines.

Les compositions selon l'invention peuvent également être utilisées à titre préventif, c'est-à-dire sur les zones de peau susceptibles d'être atteintes d'acné.

On va maintenant donner à titre d'illustration plusieurs exemples de préparation des esters aromatiques polycycliques d'antibiotiques selon l'invention ainsi que plusieurs exemples de compositions pharmaceutiques ou cosmétiques dans le traitement des dermatoses, notamment de l'acné.

EXEMPLE 1

Préparation du O-[(tétrahydro-5,6,7,8-tétraméthyl-5,5,8,8-anthracényl-2)-5-furanne-carbonyl-2]-2′-érythromycine A

Dans un ballon, sous atmosphère inerte, on dissout 5,8g (16,6mmoles) d'acide (tétrahydro-5,6,7,8-tétraméthyl-5,5,8,8-anthracényl-2)-5-furanne -2 carboxylique dans 35ml de tétrahydrofuranne anhydre; le

mélange réactionnel est refroidi à 0°C, puis on verse 2,3ml de triéthylamine et 1,6ml (16,6mmoles) de chloroformiate d'éthyle. La solution est agitée 5 minutes et on ajoute 20ml de pyridine anhydre, puis 4,9g (6,7mmoles) d'érythromycine A préalablement dissous dans 150ml de tétrahydrofuranne. Le mélange réactionnel est alors laissé sous agitation pendant 10 heures, en laissant remonter à température ambiante (chromatographie sur couche mince de gel de silice: chlorure de méthylène (90)/méthanol (10)). La solution est versée sur 60ml d'eau puis extraite à l'acétate d'éthyle. La phase organique est séchée sur sulfate de magnésium, filtrée puis concentrée sous vide partiel. Le produit brut ainsi obtenu est chromatographié sur colonne de gel de silice (H.P.L.C.) en utilisant l'éluant: acétate d'éthyle (7)/hexane (3) pour aboutir à l'isolement de 4,3g (60% de rendement) de O-[tétrahydro-5,6,7,8-tétraméthyl-5,5,8,8-anthracényl-2)-5-furanne carbonyl-2]-2′-érythromycine A

F = 136°C (hexane/acétate d'éthyle).

$[\alpha]_D^{22}$ = -57° (C = 3mg/ml dichlorométhane)

Microanalyse: $C_{60}H_{89}NO_{15}$ ; PM= 1064,4

| | C | H | N |
|---|---|---|---|
| Calculé %: | 67,71 | 8,43 | 1,32 |
| Trouvé %: | 67,51 | 8,52 | 1,25 |

R.M.N. du $^{13}C$ ($CDCl_3$, réf. interne T.M.S.).

Effets $\gamma$ négatifs en l′(-2,4ppm) et 3′ (-1,7ppm) indiquent la position de l'ester en 2′.

EXEMPLE 2

Préparation du O-[(tétrahydro-5,6,7,8-tétraméthyl-5,5,8,8-naphto [2,3-b]-furanne-yl-2)-4-benzoyl]-2′-oléandomycine

Dans un ballon, sous atmosphère inerte on dissout 300 mg (0,86 mmole) d'acide (tétrahydro-5,6,7,8-tétraméthyl-5,5,8,8-naphto[2,3-b]-furanne-2-yl)-4-benzoique dans 10 ml de tétrahydrofuranne anhydre;le mélange réactionnel est refroidi à 0°C puis on verse 0,12 ml de triéthylamine et 0,08 ml (0,86 mmole) de chloroformiate d'éthyle;la solution est agitée 1 heure et on ajoute 0,15 ml de pyridine anhydre puis 270 mg (0,35 mmole d'oléandomycine préalablement dissous dans 15 ml de tétrahydrofuranne. Le mélange réactionnel est alors laissé sous agitation pendant 10 heures en laissant remonter à température ambiante (chromatographie sur couche mince de gel de silice : chlorure de méthylène/méthanol 10 %). La solution est versée sur 25 ml d'eau puis extraite à l'acétate d'éthyle. La phase organique est séchée sur sulfate de magnésium, filtrée puis concentrée sous vide partiel. Le produit brut ainsi obtenu est chromatographié sur colonne de gel de silice (H.P.L.C.) en utilisant l'éluant : acétate d'éthyle (7)/hexane (3) pour aboutir à l'isolement de 192 mg (55 % de rendement) de O-[(tétrahydro-5,6,7,8-tétraméthyl-5,5,8,8-naphto [2,3-b]-furanne-yl-2)-4-benzoyl]-2′-oléandomycine.

$[\alpha]_D^{20}$ = -41° (C = 1 mg/ml, dichlorométhane)

F = 134°C (acétate d'éthyle/heptane).

R.M.N. du $^{13}C$ ($CDCl_3$, réf. interne T.M.S.)

Effets $\gamma$ négatifs en 1′ (-2 ppm) et 3′(-2,2 ppm) indiquent la position de l'ester en 2′.

Les autres composés énumérés aux pages 5 et 6 peuvent être préparés selon le même mode opératoire que celui décrit aux exemples 1 et 2.

COMPOSITIONS PHARMACEUTIQUES ET COSMETIQUES

A. GELS POUR LE TRAITEMENT TOPIQUE DE L'ACNE

1-
- Hydroxypropyl cellulose.................... 1g
- Butylhydroxytoluène........................ 0,05g
- O-[(tétrahydro-5,6,7,8-tétraméthyl-5,5,8,8-anthracényl-2)-5-furanne-carbonyl-2]-2'-érythromycine A............................ 0,3g
- Isopropanol q.s.p.......................... 100g

2-
- Hydroxypropyl cellulose .................... 1g
- Butylhydroxytoluène ....................... 0,05g
- O-[(tétrahydro-5,6,7,8-tétraméthyl-5,5,8,8-naphto-[2,3-b]-furanne-yl-2)-4-benzoyl]-2'-oléandomycine ............................ 0,5g
- Isopropanol q.s.p ......................... 100g

B. LOTIONS POUR LE TRAITEMENT TOPIQUE DE L'ACNE

1-
- Butylhydroxytoluène ....................... 0,05g
- O-[(tétrahydro-5,6,7,8-tétraméthyl-5,5,8,8-anthracényl-2)-5-furanne-carbonyl-2]-2' érythromycine A .......................... 1g
- Triglycérides d'acides gras en $C_8$-$C_{12}$ q.s.p........................... 100g

2-
- Butylhydroxytoluène ....................... 0,05g
- O-[(tétrahydro-5,6,7,8-tétraméthyl-5,5,8,8-naphto[2,3-b]-furanne-yl-2)-4-benzoyl]-2'-

oléandomycine ............................ 1g
- Isopropanol ............................. 30g
- Triglycérides d'acides gras en $C_8$-$C_{12}$ q.s.p. ......................... 100g

C. <u>STICKS POUR LE TRAITEMENT TOPIQUE DE L'ACNE</u>

1- - Vaseline blanche .......................... 52g
- Huile de vaseline ......................... 15g
- Paraffine raffinée ........................ 32g
- O-[(tétrahydro-5,6,7,8-tétraméthyl-5,5,8,8-anthracényl-2)-5-furanne-carbonyl-2]-2'-érythromycine A ......................... 1g

2- - Vaseline blanche .......................... 52g
- Huile de vaseline ......................... 15g
- Paraffine raffinée ........................ 32g
- O-[(tétrahydro-5,6,7,8-tétraméthyl-5,5,8,8-naphto[2,3-b]-furanne-yl-2)-4-benzoyl]-2'-oléandomycine ............................ 1g

**Revendications**

**Revendications pour les Etats contractants suivants : AT, BE, CH, LI, DE, FR, GB, GR, IT, NL, SE**

**1.** Esters aromatiques polycycliques de macrolides et de lincosamides correspondant à la formule suivante:

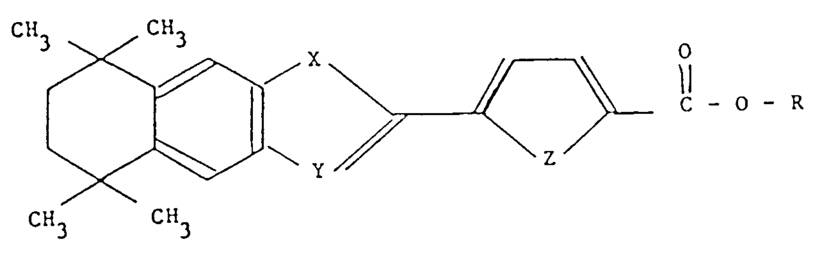
(I)

dans laquelle:

R représente un radical dérivé d'un macrolide ou d'un lincosamide,

Y représente CH ou un atome d'azote

- <u>quand Y</u> représente CH , X représente un radical vinylène (-CH=CH-), un atome d'oxygène, un atome de soufre ou $NR_1$ ,
- <u>quand Y</u> représente un atome d'azote, X représente un atome d'oxygène, un atome de soufre ou NH , et

Z représente un radical vinylène, un atome d'oxygène, un atome de soufre ou $NR_2$ ,

$R_1$ et $R_2$ représentant un atome d'hydrogène ou un radical méthyle, et les mélanges et sels

desdits esters.

2. Composés selon la revendication 1, caractérisés par le fait que le macrolide est choisi parmi l'érythromycine A, la roxythromycine, l'oléandomycine, la josamycine et les spiramycines (I), (II) et (III).

3. Composés selon la revendication 1, caractérisés par le fait que le lincosamide est choisi parmi la lincomycine et la clindamycine.

4. Composés selon la revendication 1 ou 2, caractérisés par le fait que les esters d'érythromycine A sont en position 2′.

5. Composés selon la revendication 1 ou 2, caractérisés par le fait que les esters de roxythromycine sont en position 2′.

6. Composés selon la revendication 1 ou 2, caractérisés par le fait que les esters d'oléandomycine sont en position 2′ et/ou 4″ et leurs mélanges.

7. Composés selon la revendication 1 ou 2, caractérisés par le fait que les esters de josamycine sont en position 9 et/ou 2′ et leurs mélanges.

8. Composés selon la revendication 1 ou 2, caractérisés par le fait que les esters de spiramycines (I), (II) et (III) sont en position 2' et/ou 4'' et leurs mélanges.

9. Composés selon la revendication 1 ou 3, caractérisés par le fait que les esters de lincomycine et de clindamycine sont en position 3 ou sous forme de mélanges avec les esters en position 2, 4 et 7 de lincomycine et avec les esters en position 2 et 4 de clindamycine.

10. Composés selon les revendications 1 à 9, caractérisés par le fait qu'ils sont pris dans le groupe constitué par:
O-[(tétrahydro-5,6,7,8-tétraméthyl-5,5,8,8-anthracényl-2)-5furanne-carbonyl-2]-2'-érythromycine A,
O-[(tétrahydro-5,6,7,8-tétraméthyl-5,5,8,8-anthracényl-2)-5furanne-carbonyl-2]-2'-oléandomycine,
O-[(tétrahydro-5,6,7,8-tétraméthyl-5,5,8,8-anthracényl-2)-5furanne-carbonyl-2]-3-clindamycine,
O-[(tétrahydro-5,6,7,8-tétraméthyl-5,5,8,8-anthracényl-2)-5furanne-carbonyl-2]-2'-roxythromycine,
O-[(tétrahydro-5,6,7,8-tétraméthyl-5,5,8,8-anthracényl-2)-5furanne-carbonyl-2]-9-josamycine et son isomère en 2',
O-[(tétrahydro-5,6,7,8-tétraméthyl-5,5,8,8-naphto [2,3-b] furanne-yl-2)-4- benzoyl]-2' -érythromycine A,
O-[(tétrahydro-5,6,7,8-tétraméthyl-5,5,8,8-naphto[2,3-b] thiényl-2)-4- benzoyl]-2'-érythromycine A,
O-[(tétrahydro-2,3,5,6-tétraméthyl-1,1,4,4-1H-benz[f] indolyl-2)-4- benzoyl]-2'-roxythromycine,
O-[ (méthyl-1-tétrahydro-2,3,5,6- tétraméthyl-1,1,4,4,-1H-benz[f]indolyl-2)-4- benzoyl]-9-josamycine et son isomère en 2',
O-[(tétrahydro-5,6,7,8-tétraméthyl-5,5,8,8-naphto[2,3-d] oxazolyl-2)-4- benzoyl]-2'-oléandomycine,
O-[(tétrahydro-5,6,7,8-tétraméthyl-5,5,8,8-naphto[2,3-d] imidazolyl-2)-4- benzoyl]-2' -érythromycine A,
O-[(tétrahydro-5,6,7,8-tétraméthyl-5,5,8,8-anthracényl-2)-4-benzoyl]-3 -lincomycine,
O-[(tétrahydro-5,6,7,8-tétraméthyl-5,5,8,8-anthracényl-2)-5-thiophène-carbonyl-2]-2'-spiramycine (I), (II) et (III).
O-[(tétrahydro-5,6,7,8-tétraméthyl-5,5,8,8-naphto[2,3-b] furanne-yl-2)-4-benzoyl]-2'-oléandomycine.

11. Procédé de préparation des esters aromatiques polycycliques selon l'une quelconque des revendications 1 à 10, caractérisé par le fait qu'il consiste à faire réagir en milieu solvant organique anhydre, un excès d'un anhydride mixte de formule:

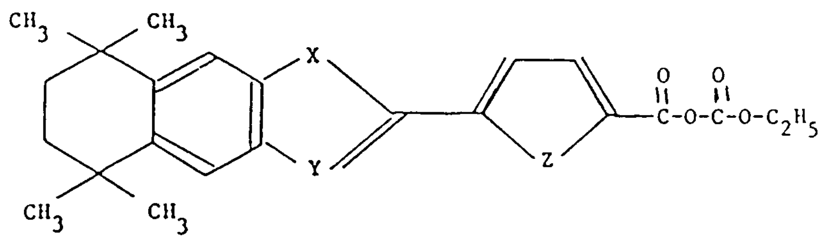

dans laquelle:

X, Y et Z ont les mêmes significations que celles données à la revendication 1, avec un macrolide ou lincosamide, sous forme de base, en présence d'une base organique ou minérale.

**12.** Procédé selon la revendication 11, caractérisé par le fait que le solvant organique anhydre est le tétrahydrofuranne seul ou en mélange avec de la pyridine.

**13.** Procédé selon la revendication 11, caractérisé par le fait que la base organique ou minérale est la pyridine et/ou l'hydrogénocarbonate de sodium.

**14.** Composition pharmaceutique ou cosmétique pour le traitement de diverses dermatoses, notamment de l'acné, caractérisée par le fait qu'elle contient dans un véhicule anhydre, en tant que composé actif, au moins un ester aromatique polycyclique de macrolides ou de lincosamides selon l'une quelconque des revendications 1 à 10 ou obtenu selon l'une quelconque des revendications 11 à 13.

**15.** Composition selon la revendication 14, caractérisée par le fait qu'elle contient de 0,001 à 10% en poids et de préférence de 0,01 à 1% de composé actif.

**16.** Composition selon l'une quelconque des revendications 14 et 15, caractérisée par le fait que le véhicule est l'acétone, l'alcool isopropylique, les triglycérides d'acides gras, les esters d'alkyle en $C_1$-$C_4$ d'acides à courte chaîne, les éthers de polytétrahydrofuranne les silicones et leurs mélanges.

**17.** Composition selon l'une quelconque des revendications 14 à 16, caractérisée par le fait qu'elle contient en outre un agent épaississant tel qu'un dérivé de cellulose en une proportion de 0,5 à 20% en poids par rapport au poids total de la composition.

**18.** Composition selon l'une quelconque des revendications 14 à 17, caractérisée par le fait qu'elle contient également un agent anti-oxydant, un agent conservateur, un parfum, un colorant ou un autre agent anti-acnéique.

**Revendications pour l'Etat contractant suivant : ES**

**1.** Composition cosmétique pour les soins de la peau, caractérisée par le fait qu'elle contient dans un véhicule anhydre, en tant que composé actif, au moins un ester aromatique polycyclique de macrolides ou de lincosamides répondant à la formule suivante :

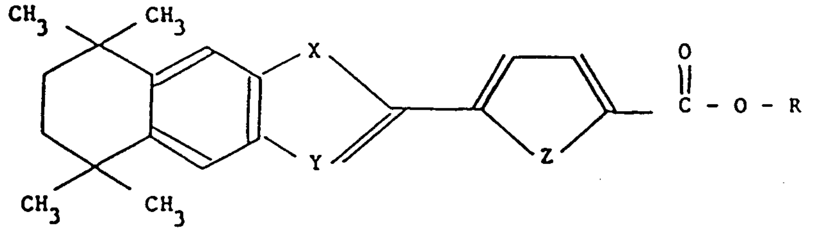

(I)

- quand Y représente un atome d'azote, X représente un atome d'oxygène, un atome de soufre ou NH , et

Z représente un radical vinylène, un atome d'oxygène, un atome de soufre ou $NR_2$ ,
$R_1$ et $R_2$ représentant un atome d'hydrogène ou un radical méthyle, et les mélanges et sels desdits esters.

**2.** Composition selon la revendication 1, caractérisée par le fait que le macrolide est choisi parmi l'érythromycine A, la roxythromycine, l'oléandomycine, la josamycine et les spiramycines (I), (II), et (III).

**3.** Composition selon la revendication 1, caractérisée par le fait que le lincosamide est choisi parmi la lincomycine et la clindamycine.

**4.** Composition selon la revendication 1 ou 2, caractérisée par le fait que les esters d'érythromycine A sont en position 2'.

**5.** Composition selon la revendication 1 ou 2, caractérisée par le fait que les esters d'oléandomycine sont en position 2'.

**6.** Composition selon la revendication 1 ou 2, caractérisée par le fait que les esters d'oléandomycine sont en position 2' et/ou 4'' et leurs mélanges.

**7.** Composition selon la revendication 1 ou 2, caractérisée par le fait que les esters de josamycine sont en position 9 et/ou 2' et leurs mélanges.

**8.** Composition selon la revendication 1 ou 2, caractérisée par le fait que les esters de spiramycines (I), (II) et (III) sont en position 2' et/ou 4'' et leurs mélanges.

**9.** Composition selon la revendication 1 ou 3, caractérisée par le fait que les esters de lincomycine et de clindamycine sont en position 3 ou sous forme de mélanges avec les esters en position 2, 4 et 7 de lincomycine et avec les esters en position 2 et 4 de clindamycine.

**10.** Composition selon les revendications 1 à 9, caractérisée par le fait que le composé actif est pris dans le groupe constitué par :
O-[(tétrahydro-5,6,7,8-tétraméthyl-5,5,8,8-anthracényl-2)-5furanne-carbonyl-2]-2'-érythromycine A,
O-[(tétrahydro-5,6,7,8-tétraméthyl-5,5,8,8-anthracényl-2)-5furanne-carbonyl-2]-2'-oléandomycine,
O-[(tétrahydro-5,6,7,8-tétraméthyl-5,5,8,8-anthracényl-2)-5furanne-carbonyl-2]-3-clindamycine,
O-[(tétrahydro-5,6,7,8-tétraméthyl-5,5,8,8-anthracényl-2)-5furanne-carbonyl-2]-2'-roxythromycine,
O-[(tétrahydro-5,6,7,8-tétraméthyl-5,5,8,8-anthracényl-2)-5furanne-carbonyl-2]-9-josamycine et son isomère en 2',
O-[(tétrahydro-5,6,7,8-tétraméthyl-5,5,8,8-naphto [2,3-b] furanne-yl-2)-4- benzoyl]-2' -érythromycine A,
O-[(tétrahydro-5,6,7,8-tétraméthyl-5,5,8,8-naphto[2,3-b] thiényl-2)-4- benzoyl]-2'-érythromycine A,
O-[(tétrahydro-2,3,5,6-tétraméthyl-1,1,4,4-1H-benz[f] indolyl-2)-4- benzoyl]-2'-roxythromycine,
O-[ (méthyl-1-tétrahydro-2,3,5,6- tétraméthyl-1,1,4,4,-1H-benz[f]indolyl-2)-4- benzoyl]-9-josamycine et son isomère en 2',
O-[(tétrahydro-5,6,7,8-tétraméthyl-5,5,8,8-naphto[2,3-d] oxazolyl-2)-4- benzoyl]-2'-oléandomycine,
O-[(tétrahydro-5,6,7,8-tétraméthyl-5,5,8,8-naphto[2,3-d] imidazolyl-2)-4- benzoyl]-2' -érythromycine A,
O-[(tétrahydro-5,6,7,8-tétraméthyl-5,5,8,8-anthracényl-2)-4-benzoyl]-3-lincomycine,
O-[(tétrahydro-5,6,7,8-tétraméthyl-5,5,8,8-anthracényl-2)-5-thiophène-carbonyl-2]-2'-spiramycine (I), (II) et (III).
O-[(tétrahydro-5,6,7,8-tétraméthyl-5,5,8,8-naphto[2,3-b] furanne-yl-2)-4-benzoyl]-2'-oléandomycine.

**11.** Composition selon l'une quelconque des revendications 1 à 10 caractérisée par le fait qu'elle contient de 0,001 à 10% en poids et de préférence de 0,01 à 1% de composé actif.

**12.** Composition selon l'une quelconque des revendications 1 à 11 caractérisée par le fait que le véhicule est l'acétone, l'alcool isopropylique, les triglycérides d'acides gras, les esters d'alkyle en $C_1$-$C_4$ d'acides à courte chaîne, les éthers de polytétrahydrofuranne les silicones et leurs mélanges.

**13.** Composition selon l'une quelconque des revendications 1 à 12 caractérisée par le fait qu'elle contient en outre un agent épaississant tel qu'un dérivé de cellulose en une proportion de 0,5 à 20% en poids par rapport au poids total de la composition.

**14.** Composition selon l'une quelconque des revendications 1 à 13, caractérisée par le fait qu'elle contient également un agent anti-oxydant, un agent conservateur, un parfum, un colorant ou un autre agent anti-acnéique.

**15.** Procédé de préparation des esters aromatiques polycycliques de formule (I) selon la revendication 1, caractérisé par le fait qu'il consiste à faire réagir en milieu solvant organique anhydre, un excès d'un anhydride mixte de formule :

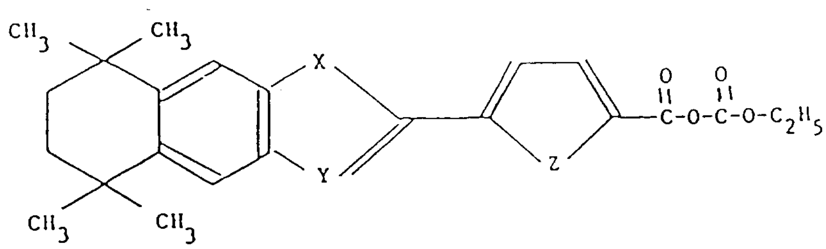

dans laquelle:

X, Y et Z ont les mêmes significations que celles doneés à la revendication 1, avec un macrolide ou lincosamide, sous forme de base, en présence d'une base organique ou minérale.

**16.** Procédé selon la revendication 15, caractérisé par le fait que le solvant organique anhydre est le tétrahydrofuranne seul ou en mélange avec de la pyridine.

**17.** Procédé selon la revendication 15, caractérisé par le fait que la base organique ou minérale est la pyridine et/ou l'hydrogénocarbonate de sodium.

**Claims**

**Claims for the following Contracting States : AT, BE, CH, LI, DE, FR, GB, GR, IT, NL, SE**

**1.** Polycyclic aromatic esters of macrolides and of lincosamides corresponding to the following formula:

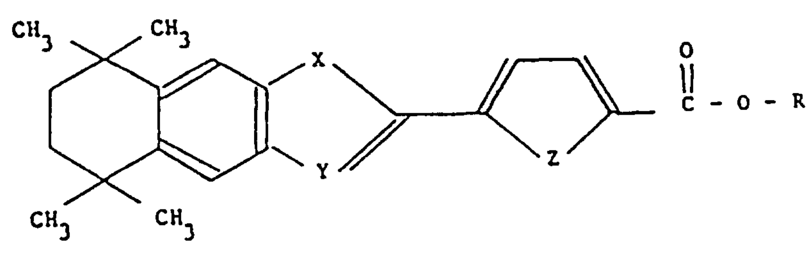

(I)

in which:

R represents a radical derived from a macrolide or lincosamide,

Y represents CH or a nitrogen atom,

- when Y represents CH, X represents a vinylene radical (-CH=CH-), an oxygen atom, a sulphur atom or $NR_1$,
- when Y represents a nitrogen atom, X represents an oxygen atom, a sulphur atom or NH, and

Z represents a vinylene radical, an oxygen atom, a sulphur atom or $NR_2$,

$R_1$ and $R_2$ represent a hydrogen atom or a methyl radical,

and the mixtures and salts of the said esters.

2. Compounds according to Claim 1, characterized in that the macrolide is chosen from erythromycin A, roxithromycin, oleandomycin, josamycin and spiramycins I, II and III.

3. Compounds according to Claim 1, characterized in that the lincosamide is chosen from lincomycin and clindamycin.

4. Compounds according to Claim 1 or 2, characterized in that the esters of erythromycin A are at position 2'.

5. Compounds according to Claim 1 or 2, characterized in that the esters of roxithromycin are at position 2'.

6. Compounds according to Claim 1 or 2, characterized in that the esters of oleandomycin are at position(s) 2' and/or 4'' and mixtures thereof.

7. Compounds according to Claim 1 or 2, characterized in that the esters of josamycin are at position(s) 9 and/or 2' and mixtures thereof.

8. Compounds according to Claim 1 or 2, characterized in that the esters of spiramycins I, II and III are at position(s) 2' and/or 4'' and mixtures thereof.

9. Compounds according to Claim 1 or 3, characterized in that the esters of lincomycin and of clindamycin are at position 3, or in the form of mixtures with the esters at positions 2, 4 and 7 of lincomycin and with the esters at positions 2 and 4 of clindamycin.

10. Compounds according to Claims 1 to 9, characterized in that they are taken from the group consisting of:
2'-O-[5-(5,6,7,8-tetrahydro-5,5,8,8-tetramethyl-2-anthracenyl)-2-furancarbonyl]erythromycin A,
2'-O-[5-(5,6,7,8-tetrahydro-5,5,8,8-tetramethyl-2-anthracenyl)-2-furancarbonyl]oleandomycin,
3-O-[5-(5,6,7,8-tetrahydro-5,5,8,8-tetramethyl-2-anthracenyl)-2-furancarbonyl]clindamycin,
2'-O-[5-(5,6,7,8-tetrahydro-5,5,8,8-tetramethyl-2-anthracenyl)-2-furancarbonyl]roxithromycin,
9-O-[5-(5,6,7,8-tetrahydro-5,5,8,8-tetramethyl-2-anthracenyl)-2-furancarbonyl]josamycin and its isomer at position 2',
2'-O-[4-(5,6,7,8-tetrahydro-5,5,8,8-tetramethyl-naphtho[2,3-b]furan-2-yl)benzoyl]erythromycin A,
2'-O-[4-(5,6,7,8-tetrahydro-5,5,8,8-tetramethyl-naphtho[2,3-b]thien-2-yl)benzoyl]erythromycin A,
2'-O-[4-(2,3,5,6-tetrahydro-1,1,4,4-tetramethyl-1H-benz[f]indol-2-yl)benzoyl]roxithromycin,
9-O-[4-(1-methyl-2,3,5,6-tetrahydro-1,1,4,4-tetramethyl-1H-benz[f]indol-2-yl)benzoyl]josamycin and its isomer at position 2',
2'-O-[4-(5,6,7,8-tetrahydro-5,5,8,8-tetramethyl-naphtho[2,3-d]oxazol-2-yl)benzoyl]oleandomycin,
2'-O-[4-(5,6,7,8-tetrahydro-5,5,8,8-tetramethyl-naphtho[2,3-d]imidazol-2-yl)benzoyl]erythromycin A,
3-O-[4-(5,6,7,8-tetrahydro-5,5,8,8-tetramethyl-2-anthracenyl)benzoyl]lincomycin,
2'-O-[5-(5,6,7,8-tetrahydro-5,5,8,8-tetramethyl-2-anthracenyl)-2-thiophenecarbonyl]spiramycin I, II and III,
2'-O-[4-(5,6,7,8-tetrahydro-5,5,8,8-tetramethyl-naphtho[2,3-b]furan-2-yl)benzoyl]oleandomycin.

11. Process for preparing the polycyclic aromatic esters according to any one of Claims 1 to 10, characterized in that it consists in reacting, in an anhydrous organic solvent medium, an excess of a mixed anhydride of formula:

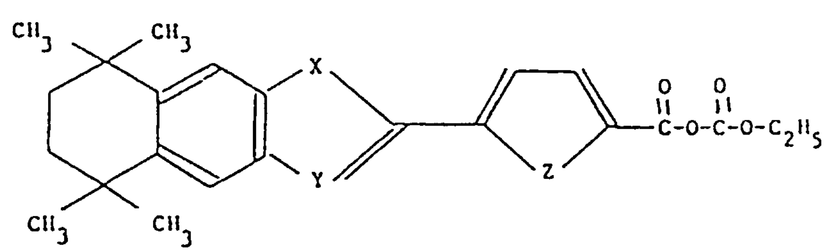

in which:

X, Y and Z have the same meanings as those given in Claim 1, with a macrolide or lincosamide, in base form, in the presence of an organic or inorganic base.

**12.** Process according to Claim 11, characterized in that the anhydrous organic solvent is tetrahydrofuran, alone or mixed with pyridine.

**13.** Process according to Claim 11, characterized in that the organic or inorganic base is pyridine and/or sodium hydrogen carbonate.

**14.** Pharmaceutical or cosmetic composition for the treatment of various dermatoses, in particular acne, characterized in that it contains, in an anhydrous vehicle, as active compound, at least one polycyclic aromatic ester of macrolides or of lincosamides according to any one of Claims 1 to 10, or obtained according to any one of Claims 11 to 13.

**15.** Composition according to Claim 14, characterized in that it contains from 0.001 to 10% by weight, and preferably from 0.01 to 1%, of active compound.

**16.** Composition according to either of Claims 14 and 15, characterized in that the vehicle is acetone, isopropyl alcohol, fatty acid triglycerides, $C_1$-$C_4$ alkyl esters of short-chain acids, polytetrahydrofuran ethers, silicones and mixtures thereof.

**17.** Composition according to any one of Claims 14 to 16, characterized in that it contains, in addition, a thickening agent such as a cellulose derivative in a proportion of 0.5 to 20% by weight relative to the total weight of the composition.

**18.** Composition according to any one of Claims 14 to 17, characterized in that it also contains an antioxidant, a preservative, a perfume, a colorant or another anti-acne agent.

**Claims for the following Contracting State : ES**

**1.** Cosmetic composition for skin care, characterized in that it contains, in an anhydrous vehicle, as active compound, at least one polycyclic aromatic ester of macrolides or of lincosamides corresponding to the following formula:

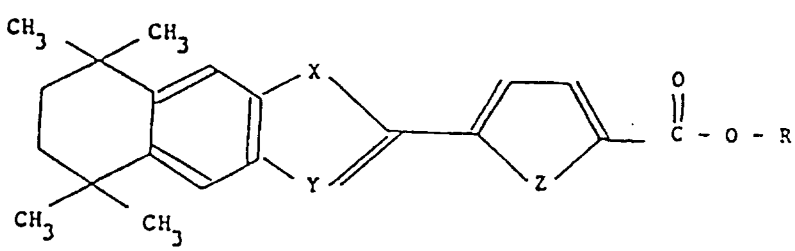

(I)

in which:

R represents a radical derived from a macrolide or lincosamide,

Y represents CH or a nitrogen atom,

- when Y represents CH, X represents a vinylene radical (-CH=CH-), an oxygen atom, a sulphur atom or $NR_1$,
- when Y represents a nitrogen atom, X represents an oxygen atom, a sulphur atom or NH, and

Z represents a vinylene radical, an oxygen atom, a sulphur atom or $NR_2$,

$R_1$ and $R_2$ represent a hydrogen atom or a methyl radical,

and the mixtures and salts of the said esters.

**2.** Composition according to Claim 1, characterized in that the macrolide is chosen from erythromycin A, roxithromycin, oleandomycin, josamycin and spiramycins I, II and III.

3. Composition according to Claim 1, characterized in that the lincosamide is chosen from lincomycin and clindamycin.

4. Composition according to Claim 1 or 2, characterized in that the esters of erythromycin A are at position 2'.

5. Composition according to Claim 1 or 2, characterized in that the esters of roxithromycin are at position 2'.

6. Composition according to Claim 1 or 2, characterized in that the esters of oleandomycin are at position(s) 2' and/or 4'' and mixtures thereof.

7. Composition according to Claim 1 or 2, characterized in that the esters of josamycin are at position(s) 9 and/or 2' and mixtures thereof.

8. Composition according to Claim 1 or 2, characterized in that the esters of spiramycins I, II and III are at position(s) 2' and/or 4'' and mixtures thereof.

9. Composition according to Claim 1 or 3, characterized in that the esters of lincomycin and of clindamycin are at position 3, or in the form of mixtures with the esters at positions 2, 4 and 7 of lincomycin and with the esters at positions 2 and 4 of clindamycin.

10. Composition according to Claims 1 to 9, characterized in that the active compound is taken from the group consisting of:
2'-O-[5-(5,6,7,8-tetrahydro-5,5,8,8-tetramethyl-2-anthracenyl)-2-furancarbonyl]erythromycin A,
2'-O-[5-(5,6,7,8-tetrahydro-5,5,8,8-tetramethyl-2-anthracenyl)-2-furancarbonyl]oleandomycin,
3-O-[5-(5,6,7,8-tetrahydro-5,5,8,8-tetramethyl-2-anthracenyl)-2-furancarbonyl]clindamycin,
2'-O-[5-(5,6,7,8-tetrahydro-5,5,8,8-tetramethyl-2-anthracenyl)-2-furancarbonyl]roxithromycin,
9-O-[5-(5,6,7,8-tetrahydro-5,5,8,8-tetramethyl-2-anthracenyl)-2-furancarbonyl]josamycin and its isomer at position 2',
2'-O-[4-(5,6,7,8-tetrahydro-5,5,8,8-tetramethyl-naphtho[2,3-b]furan-2-yl)benzoyl]erythromycin A,
2'-O-[4-(5,6,7,8-tetrahydro-5,5,8,8-tetramethyl-naphtho[2,3-b]thien-2-yl)benzoyl]erythromycin A,
2'-O-[4-(2,3,5,6-tetrahydro-1,1,4,4-tetramethyl-1H-benz[f]indol-2-yl)benzoyl]roxithromycin,
9-O-[4-(1-methyl-2,3,5,6-tetrahydrol-1,1,4,4-tetramethyl-1H-benz[f]indol-2-yl)benzoyl]josamycin and its isomer at position 2',
2'-O-[4-(5,6,7,8-tetrahydro-5,5,8,8-tetramethyl-naphtho[2,3-d]oxazol-2-yl)benzoyl]oleandomycin,
2'-O-[4-(5,6,7,8-tetrahydro-5,5,8,8-tetramethyl-naphtho[2,3-d]imidazol-2-yl)benzoyl]erythromycin A,
3-O-[4-(5,6,7,8-tetrahydro-5,5,8,8-tetramethyl-2-anthracenyl)benzoyl]lincomycin,
2'-O-[5-(5,6,7,8-tetrahydro-5,5,8,8-tetramethyl-2-anthracenyl)-2-thiophenecarbonyl]spiramycin I, II and III,
2'-O-[4-(5,6,7,8-tetrahydro-5,5,8,8-tetramethyl-naphtho[2,3-b]furan-2-yl)benzoyl]oleandomycin.

11. Composition according to any one of Claims 1 to 10, characterized in that it contains from 0.001 to 10% by weight, and preferably from 0.01 to 1%, of active compound.

12. Composition according to any one of Claims 1 to 11, characterized in that the vehicle is acetone, isopropyl alcohol, fatty acid triglycerides, $C_1$-$C_4$ alkyl esters of short-chain acids, polytetrahydrofuran ethers, silicones and mixtures thereof.

13. Composition according to any one of Claims 1 to 12, characterized in that it contains, in addition, a thickening agent such as a cellulose derivative in a proportion of 0.5 to 20% by weight relative to the total weight of the composition.

14. Composition according to any one of Claims 1 to 13, characterized in that it also contains an antioxidant, a preservative, a perfume, a colorant or another anti-acne agent.

15. Process for preparing the polycyclic aromatic esters of formula (I) according to Claim 1, characterized in that it consists in reacting, in an anhydrous organic solvent medium, an excess of a mixed anhydride

of formula:

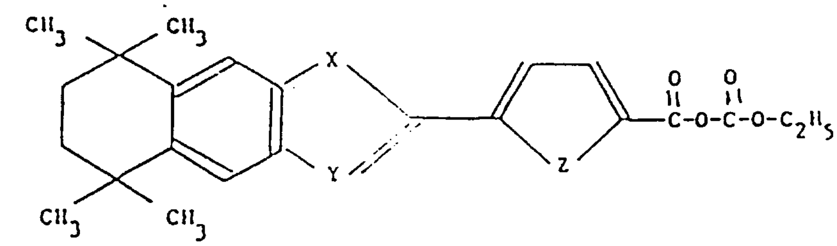

in which:

X, Y and Z have the same meanings as those given in Claim 1, with a macrolide or lincosamide, in base form, in the presence of an organic or inorganic base.

**16.** Process according to Claim 15, characterized in that the anhydrous organic solvent is tetrahydrofuran, alone or mixed with pyridine.

**17.** Process according to Claim 15, characterized in that the organic or inorganic base is pyridine and/or sodium hydrogen carbonate.

**Patentansprüche**

**Patentansprüche für folgende Vertragsstaaten : AT, BE, CH, LI, DE, FR, GB, GR, IT, NL, SE**

**1.** Aromatische polycyclische Ester vom Makrolid- und Lincosamidtyp, welche der folgenden Formel entsprechen:

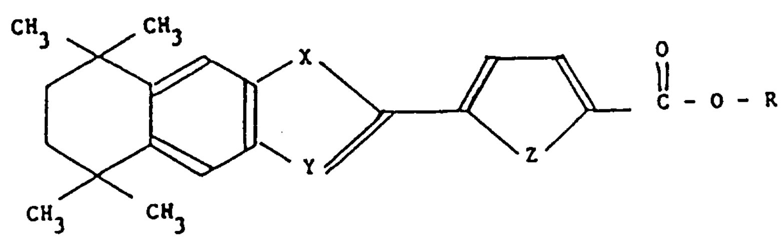 (I)

worin

R einen von einem Makrolid oder Lincosamid abgeleiteten Rest,

Y CH oder ein Stickstoffatom bedeuten,

unter der Bedingung, daß dann, wenn Y CH bedeutet, X eine Vinylengruppe (-CH = CH-), ein Sauerstoffatom, ein Schwefelatom oder $NR_1$ bedeutet, oder in dem Fall, daß Y ein Stickstoffatom bedeutet, X ein Sauerstoffatom, ein Schwefelatom oder NH bedeutet, und

Z eine Vinylengruppe, ein Sauerstoffatom, ein Schwefelatom oder $NR_2$ bedeutet, wobei

$R_1$ und $R_2$ ein Wasserstoffatom oder eine Methylgruppe bedeuten,

sowie Mischungen und Salze der genannten Ester.

**2.** Verbindungen gemäß Anspruch 1, dadurch gekennzeichnet, daß das Makrolid aus Erythromycin A, Roxythromycin, Oleandomycin, Josamycin und den Spiramycinen (I), (II) sowie (III) ausgewählt ist.

**3.** Verbindungen gemäß Anspruch 1, dadurch gekennzeichnet, daß das Lincosamid unter Lincomycin und Clindamycin ausgewählt ist.

**4.** Verbindungen gemäß Anspruch 1 oder 2, dadurch gekennzeichnet, daß die Ester von Erythromycin A in der 2'-Stellung konstituiert sind.

5. Verbindungen gemäß den Ansprüchen 1 oder 2, dadurch gekennzeichnet, daß die Ester von Roxythromycin in der 2'-Stellung. konstituiert sind.

6. Verbindungen gemäß Anspruch 1 oder 2, dadurch gekennzeichnet, daß die Ester von Oleandomycin in der 2'-Stellung und/oder 4''-Stellung konstituiert sind, sowie deren Gemische.

7. Verbindungen gemäß Anspruch 1 oder 2, dadurch gekennzeichnet, daß die Ester von Josamycin in der 9-Stellung und/oder 2'-Stellung konstituiert sind, sowie deren Gemische.

8. Verbindungen gemäß Anspruch 1 oder 2, dadurch gekennzeichnet, daß die Ester von Spiramycin (I), (II) und (III) in der 2'-Stellung und/oder 4''-Stellung konstituiert sind, sowie deren Gemische.

9. Verbindungen gemäß Anspruch 1 oder 3, dadurch gekennzeichnet, daß die Ester von Lincomycin und Clindamycin in der 3-Stellung konstituiert sind oder in Form der Mischungen zusammen mit den Estern in der 2-, 4- und 7-Stellung von Lincomycin sowie mit den Estern in der 2- und 4-Stellung von Clindamycin vorliegen.

10. Verbindungen gemäß den Ansprüchen 1 bis 9, dadurch gekennzeichnet, daß sie aus der folgenden Gruppe ausgewählt sind:
- 2'-Erythromycin-A-O-[(5,6,7,8-tetrahydro-5,5,8,8-tetramethyl-2-anthracenyl)-5-furan-2-carbonsäureester],
- 2'-Oleandomycin-O-[(5,6,7,8-tetrahydro-5,5,8,8-tetramethyl-2-anthracenyl)-5-furan-2-carbonsäureester],
- 3-Clindamycin-O-[(5,6,7,8-tetrahydro-5,5,8,8-tetramethyl-2-anthracenyl)-5-furan-2-carbonsäureester],
- 2'-Roxythromycin-O-[(5,6,7,8-tetrahydro-5,5,8,8-tetramethyl-2-anthracenyl)-5-furan-2-carbonsäureester],
- 9-Josamycin-O-[(5,6,7,8-tetrahydro-5,5,8,8-tetramethyl-2-anthracenyl)-5-furan-2-carbonsäureester] sowie dessen 2'-Isomer,
- 2'-Erythromycin-A-O-[(5,6,7,8-tetrahydro-5,5,8,8-tetramethyl-naphtho[2,3-b]furan-2-yl)-4-benzoat],
- 2'-Erythromycin-A-O-[(5,6,7,8-tetrahydro-5,5,8,8-tetramethyl)-2-naphtho[2,3-b]thienyl)-4-benzoat],
- 2'-Roxythromycin-O-[(2,3,5,6-tetrahydro-1,1,4,4-tetramethyl-2-1H-benz[f]indolyl)-4-benzoat],
- 9-Josamycin-O-[(1-methyl-2,3,5,6-tetrahydro-1,1,4,4-tetramethyl-1H-benz[f]indolyl-2)-4-benzoat] sowie dessen 2'-Isomer,
- 2'-Oleandomycin-O-[(5,6,7,8-tetrahydro-5,5,8,8-tetramethyl-2-naphtho[2,3-d]oxazolyl)-4-benzoat],
- 2'-Erythromycin-A-O-[(5,6,7,8-tetrahydro-5,5,8,8-tetramethyl-2-naphtho[2,3-d]imidazolyl)-4-benzoat],
- 3-Lincomycin-O-[(5,6,7,8-tetrahydro-5,5,8,8-tetramethyl-2-anthracenyl)-4-benzoat],
- 2'-Spiramycin-(I), (II) sowie (III)-O-[(5,6,7,8-tetrahydro-5,5,8,8-tetramethyl-2-anthracenyl)-5-thiophen-2-carbonsäureester],
- 2'-Oleandomycin-O-[(5,6,7,8-tetrahydro-5,5,8,8-tetramethyl-naphtho[2,3-b]furan-2-yl)-4-benzoat].

11. Verfahren zur Herstellung von polycyclischen aromatischen Estern gemäß einem der Ansprüche 1 bis 10, dadurch gekennzeichnet, daß man in wasserfreiem organischen Lösungsmittelmilieu einen Überschuß an gemischtem Anhydrid der folgenden Formel:

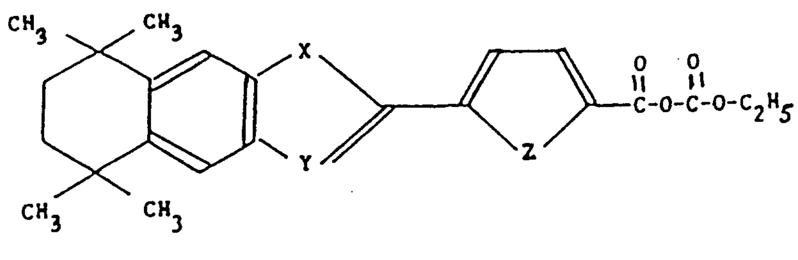

worin
X, Y und Z dieselben Bedeutungen haben, wie sie in Anspruch 1 definiert sind, zusammen mit einem

Makrolid oder Lincosamid in Form ihrer Base, in Anwesenheit einer organischen oder mineralischen Base, reagieren läßt.

**12.** Verfahren gemäß Anspruch 11, dadurch gekennzeichnet, daß das wasserfreie organische Lösungsmittel aus Tetrahydrofuran allein oder in Mischung zusammen mit Pyridin gebildet ist.

**13.** Verfahren gemäß Anspruch 11, dadurch gekennzeichnet, daß die organische oder mineralische Base in Form von Pyridin und/oder Natriumhydrogencarbonat vorliegt.

**14.** Pharmazeutische oder kosmetische Zubereitung zur Behandlung verschiedener Dermatosen, insbesondere der Akne, dadurch gekennzeichnet, daß sie in einem wasserfreien Träger als Wirkstoff mindestens einen polycyclischen aromatischen Ester von Makroliden oder Lincosamiden gemäß einem der Ansprüche 1 bis 10 oder einen gemäß einem der Ansprüche 11 bis 13 erhältlichen Wirkstoff enthält.

**15.** Zubereitung gemäß Anspruch 14, dadurch gekennzeichnet, daß sie zwischen 0,001 und 10 Gew.-%, vorzugsweise zwischen 0,01 und 1 % Wirkstoff enthält.

**16.** Zubereitung gemäß einem der Ansprüche 14 und 15, dadurch gekennzeichnet, daß der Träger in Form von Aceton, Isopropylalkohol, Fettsäuretriglyzeriden, kurzkettigen Alkylestern von $C_1$-$C_4$-Säuren, Polytetrahydrofuranethern, Silikonen und Mischungen vorliegt.

**17.** Zubereitung gemäß einem der Ansprüche 14 bis 16, dadurch gekennzeichnet, daß sie zusätzlich ein Verdickungsmittel wie Cellulose in einem Verhältnis von 0,5 bis 20 Gew.-% in bezug auf das Gesamtgewicht der Zubereitung enthält.

**18.** Zubereitung gemäß einem der Ansprüche 14 bis 17, dadurch gekennzeichnet, daß sie zusätzlich ein Antioxidans, ein Konservierungsmittel, einen Geruchsstoff, einen Farbstoff oder noch einen weiteren Wirkstoff gegen Akne enthält.

**Patentansprüche für folgenden Vertragsstaat : ES**

**1.** Kosmetische Zubereitung zur Hautpflege, dadurch gekennzeichnet, daß sie in einem wasserfreien Träger als Wirkstoff mindestens einen aromatischen polycyclischen Ester von Makroliden oder Licosamiden enthält, welcher der folgenden Formel entspricht:

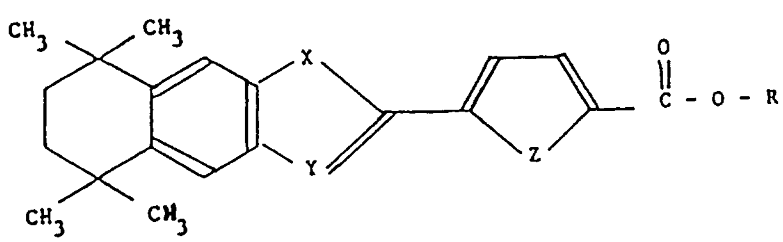

(I)

- unter der Bedingung, daß dann, wenn Y ein Stickstoffatom bedeutet, X ein Sauerstoffatom, ein Schwefelatom oder NH darstellt, und

Z eine Vinylengruppe, ein Sauerstoffatom, ein Schwefelatom oder $NR_2$ bedeutet,
wobei $R_1$ und $R_2$ ein Wasserstoffatom oder eine Methylgruppe bedeuten,
sowie Mischungen und Salze der genannten Ester.

**2.** Zubereitung gemäß Anspruch 1, dadurch gekennzeichnet, daß das Makrolid unter Erythromycin A, Roxythromycin, Oleandomycin, Josamycin und den Spiramycinen (I), (II) sowie (III) ausgewählt ist.

**3.** Zubereitung gemäß Anspruch 1, dadurch gekennzeichnet, daß das Lincosamid unter Lincomycin und Clindamycin ausgewählt ist.

4. Zubereitung gemäß Anspruch 1 oder 2, dadurch gekennzeichnet, daß die Ester von Erythromycin A in der 2'-Stellung konstituiert sind.

5. Zubereitung gemäß den Ansprüchen 1 oder 2, dadurch gekennzeichnet, daß die Ester von Roxythromycin in der 2'-Stellung konstituiert sind.

6. Zubereitung gemäß Anspruch 1 oder 2, dadurch gekennzeichnet, daß die Ester von Oleandomycin in der 2'-Stellung und/oder 4''-Stellung konstituiert sind, sowie deren Gemische.

7. Zubereitung gemäß Anspruch 1 oder 2, dadurch gekennzeichnet, daß die Ester von Josamycin in der 9-Stellung und/oder 2'-Stellung konstituiert sind, sowie deren Gemische.

8. Zubereitung gemäß Anspruch 1 oder 2, dadurch gekennzeichnet, daß die Ester von Spiramycin (I), (II) und (III) in der 2'-Stellung und/oder 4''-Stellung konstituiert sind, sowie deren Gemische.

9. Zubereitung gemäß Anspruch 1 oder 3, dadurch gekennzeichnet, daß die Ester von Lincomycin und Clindamycin in der 3-Stellung konstituiert sind oder in Form der Mischungen zusammen mit den Estern in der 2-, 4- und 7-Stellung von Lincomycin sowie mit den Estern in der 2- und 4-Stellung von Clindamycin vorliegen.

10. Zubereitung gemäß den Ansprüchen 1 bis 9, dadurch gekennzeichnet, daß sie aus der folgenden Gruppe ausgewählt sind:
   - 2'-Erythromycin-A-O-[(5,6,7,8-tetrahydro-5,5,8,8-tetramethyl-2-anthracenyl)-5-furan-2-carbonsäureester],
   - 2'-Oleandomycin-O-[(5,6,7,8-tetrahydro-5,5,8,8-tetramethyl-2-anthracenyl)-5-furan-2-carbonsäureester],
   - 3-Clindamycin-O-[(5,6,7,8-tetrahydro-5,5,8,8-tetramethyl-2-anthracenyl)-5-furan-2-carbonsäureester],
   - 2'-Roxythromycin-O-[(5,6,7,8-tetrahydro-5,5,8,8-tetramethyl-2-anthracenyl)-5-furan-2-carbonsäureester],
   - 9-Josamycin-O-[(5,6,7,8-tetrahydro-5,5,8,8-tetramethyl-2-anthracenyl)-5-furan-2-carbonsäureester] sowie dessen 2'-Isomer,
   - 2'-Erythromycin-A-O-[(5,6,7,8-tetrahydro-5,5,8,8-tetramethyl-naphtho[2,3-b]furan-2-yl)-4-benzoat],
   - 2'-Erythromycin-A-O-[(5,6,7,8-tetrahydro-5,5,8,8-tetramethyl)-2-naphtho[2,3-b]thienyl)-4-benzoat],
   - 2'-Roxythromycin-O-[(2,3,5,6-tetrahydro-1,1,4,4-tetramethyl-2-1H-benz[f]indolyl)-4-benzoat],
   - 9-Josamycin-O-[(1-methyl-2,3,5,6-tetrahydro-1,1,4,4-tetramethyl-1H-benz[f]indolyl-2)-4-benzoat] sowie dessen 2'-Isomer,
   - 2'-Oleandomycin-O-[(5,6,7,8-tetrahydro-5,5,8,8-tetramethyl-2-naphtho[2,3-d]oxazolyl)-4-benzoat],
   - 2'-Erythromycin-A-O-[(5,6,7,8-tetrahydro-5,5,8,8-tetramethyl-2-naphtho[2,3-d]imidazolyl)-4-benzoat],
   - 3-Lincomycin-O-[(5,6,7,8-tetrahydro-5,5,8,8-tetramethyl-2-anthracenyl)-4-benzoat],
   - 2'-Spiramycin-(I), (II) sowie (III)-O-[(5,6,7,8-tetrahydro-5,5,8,8-tetramethyl-2-anthracenyl)-5-thiophen-2-carbonsäureester],
   - 2'-Oleandomycin-O-[(5,6,7,8-tetrahydro-5,5,8,8-tetramethyl-naphtho[2,3-b]furan-2-yl)-4-benzoat].

11. Zubereitung gemäß einem der Ansprüche 1 bis 10, dadurch gekennzeichnet, daß sie zwischen 0,001 und 10 Gew.-%, vorzugsweise zwischen 0,01 und 1 Gew.-% Wirkstoff enthält.

12. Zubereitung gemäß einem der Ansprüche 1 bis 11, dadurch gekennzeichnet, daß der Träger in Form von Aceton, Isopropylalkohol, Fettsäuretriglyzeriden, kurzkettigen Alkylestern von $C_1$-$C_4$-Säuren, Polytetrahydrofuranethern, Silikonen und Mischungen vorliegt.

13. Zubereitung gemäß einem der Ansprüche 1 bis 12, dadurch gekennzeichnet, daß sie zusätzlich ein Verdickungsmittel wie Cellulose in einem Verhältnis von 0,5 bis 20 Gew.-% in bezug auf das Gesamtgewicht der Zubereitung enthält.

14. Zubereitung gemäß einem der Ansprüche 1 bis 13, dadurch gekennzeichnet, daß sie zusätzlich ein Antioxidans, ein Konservierungsmittel, einen Geruchsstoff, einen Farbstoff oder noch einen weiteren

Wirkstoff gegen Akne enthält.

**15.** Verfahren zur Herstellung von polycyclischen aromatischen Estern der Formel (I) gemäß Anspruch 1, dadurch gekennzeichnet, daß man in wasserfreiem organischen Lösungsmittelmilieu einen Überschuß an gemischtem Anhydrid der folgenden Formel:

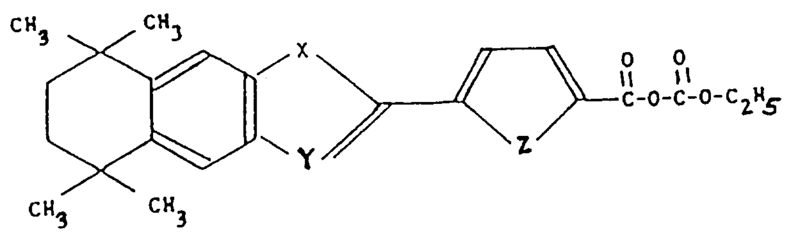

worin
X, X und Z dieselben Bedeutungen haben, wie sie in Anspruch 1 definiert sind, zusammen mit einem Makrolid oder Lincosamid in Form ihrer Base, in Anwesenheit einer organischen oder mineralischen Base reagieren läßt.

**16.** Verfahren gemäß Anspruch 15, dadurch gekennzeichnet, daß das wasserfreie organische Lösungsmittel aus Tetrahydrofuran allein oder in Mischung zusammen mit Pyridin gebildet ist.

**17.** Verfahren gemäß Anspruch 15, dadurch gekennzeichnet, daß die organische oder mineralische Base in Form von Pyridin und/oder Natriumhydrogencarbonat vorliegt.